# EUROPEAN PATENT APPLICATION

(11) **EP 2 420 492 A1**
(43) Date of publication of application: **22.02.2012**
(21) Application number: 10764470.0
(22) Date of filing: 14.04.2010
(51) Int. Cl.: C07D 213/61, C07B 61/00

(54) **METHOD FOR PRODUCING 2-HALOGENO-6-SUBSTITUTED-4-TRIFLUOROMETHYLPYRIDINE**

(30) Priority: 17.04.2009 JP 2009100749
(71) Applicant: Ishihara Sangyo Kaisha, Ltd., Osaka-shi, Osaka 550-0002 (JP)
(72) Inventor: IKEGUCHI, Masahiko, Kusatsu-shi Shiga 525-0025 (JP)
(74) Representative: HOFFMANN EITLE
(86) International application number: PCT/JP2010/056657
(87) International publication number: WO 2010/119886

(57) **Abstract**

The present invention relates to a process of a process for producing a 2-halogeno-6-substituted-4-trifluoromethylpyridine. Specifically, the present invention provides a process for producing a 2-halogeno-6-substituted-4-trifluoromethylpyrzdine represented bv the formula (I): 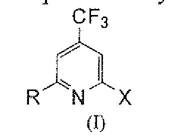 in which X is a chlorine atom, a bromine atom, or an iodine atom; R is alkyl, alkenyl, alkynyl, phenyl which may be substituted with A, benzyl which may be substituted with A, or cycloalkyl; and A is alkyl, alkoxy, a fluorine atom, or a chlorine atom,
which comprises allowing a 2,6-dihalogeno-4-tritluoroznethylpyridine represented by the formula (II): 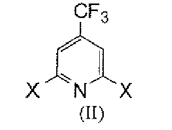 in which X is defined above,
and a Grignard reagent represented by the formula (III): RMgX, in which R and X are defined above, to react with each other in the presence of a solvent.

## Description

### TECHNICAL FIELD

The present invention relates to a process for producing a 2-halogeno-6-substituted-4-trifluoromethylpyridine which is used as an intermediate of pharmaceuticals and agricultural chemicals.

### BACKGROUND ART

Non-Patent Document 1 discloses a process for producing 2-chloro-6-methyl-4-trifluoromethylpyridine which is a compound included in the formula (I) as described later, by a two-step reaction of producing 6-methyl-4-trifluoromethyl-2(1H)pyridone from 3-cyano-6-methyl-4-trifluoromethyl-2(1H)pyridone and allowing the obtained material to react with phosphorus pentachloride and phosphorus oxychloride. However, this process is different from the process for producing a 2-halogeno-6-substituted-4-trifluoromethylpyridine of the present invention.

Non-Patent Document 2 describes a process comprising allowing 2,6-dichloropyridine and a Grignard reagent to react with each other in the presence of tetrahydrofuran, N-methylpyrrolidone and tris(acetylacetonata)iron(III), thereby selectively replacing only one of the chlorine atoms by benzyloxyhexanyl. However, not only the objective material is not the 2-halogeno-6-substituted-4-trifluoromethylpyridine, but it includes a problem that a high selectivity cannot be attained unless a Grignard reagent is slowly added dropwise.

### CITATION LIST

### Non-Patent Document

Non-Patent Document 1: J Hetrocyclic Chemistry (1969), 6(2), 223-228
Non-Patent Document 2 : Proc. Natl. Acad. Sci. USA (2004), 101, 11960-11965

### SUMMARY OF THE INVENTION

### PROBLEM TO BE SOLVED BY THE INVENTION

Although the process for producing 2-chloro-6-methyl-4-trifluoromethylpyridine was described in the above Non-Patent Document 1, not only starting materials were expensive, but also a yield thereof was insufficient. An object of the present invention is to produce a 2-halogeno-6-substituted-4-trifluoromethylpyridine including 2-chloro-6-methyl-4-trifluoromethylpyridine by an economical and simple process.

### MEANS TO SOLVE THE PROBLEM

In order to solve the above problem, the present inventors made various investigations. As a result, the inventors have found a process for producing a 2-halogeno-6-substituted-4-trifluoromethylpyridine by allowing a 2,6-dihalogeno-4-trifluoromethylpyridine and a specific Grignard reagent to react with each other in the presence of a solvent, thereby selectively replacing only one of the halogens by another substituent and accomplished the present invention. Also, the inventors found that, in the present invention, when a specific metal catalyst is used, an objective material can be obtained in a high yield even at room temperature. That is, the present invention specifically relates to a process for producing a 2-halogeno-6-substituted-4-trifluoromethylpyridine represented by the formula (I): 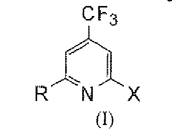 in which, X is a chlorine atom, a bromine atom, or an iodine atom; R is alkyl, alkenyl, alkynyl, phenyl which may be substituted with A, benzyl which may be substituted with A, or cycloalkyl; and A is alkyl, alkoxy, a fluorine atom, or a chlorine atom,
which comprises allowing a 2,6-dihalogeno-4-trifluoromethylpyridine represented by the formula (II): 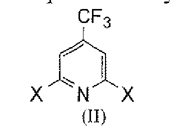 in which, X is as defined above;
and a Grignard reagent represented by the formula (III): RMgX
in which, R and X are as defined above;
to react with each other in the presence of a solvent. Furthermore, the present invention relates to a 2-halogeno-6-substituted-4-trifluoromethylpyridine represented by the formula (I-1) as described later.

The alkyl or the alkyl moiety in the alkoxy in the formula (I) may be either linear or branched, and specific examples include C₁₋₆ alkyl, such as methyl, ethyl, propyl, isopropyl, butyl, tert-butyl, pentyl, and hexyl; and the like.

The alkenyl in the formula (I) may be either linear or branched, and specific examples include C₂₋₆ alkenyl, such as vinyl, 1-propenyl, allyl, isopropenyl, 1-butenyl, 1,3-butadienyl, and 1-hexenyl; and the like.

The alkynyl in the formula (I) may be either linear or branched, and specific examples include C₂₋₆ alkynyl, such as ethynyl, 2-butynyl, 2-pentynyl, 3-methyl-1-butynyl, 2-penten-4-ynyl, and 3-hexynyl; and the like.

Examples of the cycloalkyl in the formula (I) include C₃-₆ cycloalkyl, such as cyclopropyl, cyclobutyl, cyclopentyl, and cyclohexyl; and the like.

The number of substitution of the alkyl, alkoxy, a fluorine atom or a chlorine atom contained in A in the formula (1) on phenyl or benzyl may be 1 or 2 or more, and the substitution position may be any position.

### ADVANTAGEOUS EFFECTS OF THE INVENTION

According to the production process of the present invention, the 2-halogeno-6-substituted-4-trifluoromethylpyridine can be produced by selectively replacing only one of the halogens of the 2,6-dihalogeno-4-trifluoromethylpyridine by another substituent. In addition, the objective material can be produced in a high yield by selecting tetrahydrofuran as a solvent. Furthermore, the objective material can be produced in a high yield even at room temperature by using a specific metal catalyst in combination.

### MODES FOR CARRYING OUT THE INVENTION

Hereinafter, the production process of the present invention described in detail.

A 2-halogeno-6-substituted-4-trifluorometlzylpyridine represented by the formula (I) can be produced by allowing a 2,6-dihalogeno-4-trifluoromethylpyridine represented by the formula (II) and a Grignard reagent represented by the formula (III) to react with each other in the presence of a solvent. 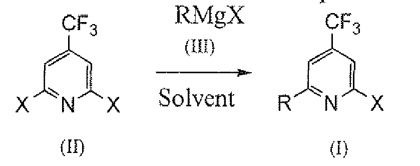 in which, X and R are as defined above.

The Grignard reagent represented by the formula (III) which is used in the present reaction can be used in an amount of 1 to 5 times by mole, and preferably 1 to 2 times by mole relative to one mole of the 2,6-dihalogeno-4-trifluoromethylpyridine represented by the formula (II). In addition, in this reaction, even when the Grignard reagent represented by the formula (III) is quickly added dropwise to the compound represented by the formula (II), the compound represented by the formula (I) is obtained with a high selectivity.

The present reaction is carried out in the presence of a solvent. Any solvent can be used as long as it is an inert solvent to the reaction. For example, one or two or more kinds of solvents can be properly selected from aromatic hydrocarbons, such as toluene and dichlorobenzene; aliphatic hydrocarbons, such as pentane, hexane, heptane, octane, and cyclohexane; ethers, such as tetrahydrofuran, 2-methyltetrahydrofuran, dioxane, diethyl ether, t-butyl methyl ether, and cyclopentyl methyl ether; polar aprotic solvents, such as hexamethylphosphoric triamide, sulfolane, dimethylacetamide, N-methylpyrrolidone, 1,2-dimethoxyethane, 1,3-dimethyl-3,4,5,6-tetrahydro-2(H)pyrimidinone, and tetramethylurea; and the like. The solvent can be used in an amount of 1 to 100 times by volume relative to the 2,6-dihalogerzo-4-trifluoromethylpyridine represented by the formula (II). Among these solvents, tetrahydrofuran is preferable in view of enhancing a reaction yield. In addition, although only tetrahydrofuran may be used as a solvent, a mixture of tetrahydrofuran with one or more kinds of other solvents may be used.

In general, the present reaction can be carried out at from -10°C to a reflux temperature of the solvent. When the reaction is carried out at a reaction temperature of preferably from -10 to 180°C, and more preferably from -10 to 120°C, it is possible to obtain the objective material in a high yield. Also, a reaction time is usually from about 0.01 to 30 hours.

In addition, it is preferable that the present reaction is carried out under an atmosphere with a small amount of moisture or oxygen, and it is preferable that the reaction is carried out in the presence of an inert gas, such as nitrogen, argon and helium.

When the present invention is carried out in the presence of a metal catalyst, the objective material can be obtained in a high yield even at the temperature which is not limited by the above reaction temperature, such as room temperature. Accordingly, it is preferable that the present reaction is carried out in the presence of a metal catalyst. Also, it is more preferable to use tetrahydrofuran as a solvent and to use a metal catalyst in combination. Examples of the metal catalyst which can be used for the present reaction include various metal catalysts. However, it is preferable to use an iron based catalyst, a palladium based catalyst, a nickel based catalyst, a zinc based catalyst, a cobalt based catalyst, a magnesium based catalyst, or a copper based catalyst. Examples of the iron based catalyst include an iron chloride such as iron(II) chloride and iron(III) chloride; an acetylacetonato iron such as bis(acetylacetonato)iron(II) and tris(acetylacetonato)iron(III); an iron oxide such as iron monoxide (FeO), diiron trioxide (Fe₂O₃), and triiron tetraoxide (Fe₃O₄); metallic iron (Fe); and the like. Examples of the palladium based catalyst include tetrakis(triphenylphosphine)palladium(0), bis(triphenylphosphine)palladium(II) chloride, palladium on carbon, and the like. Examples of the nickel based catalyst include nickel bromide, nickel chloride, (bis(diphenylphosphino)propane)dichloronickel, and the like. Examples of the zinc based catalyst include zinc bromide and the like. Examples of the cobalt based catalyst include bis(acetylacetonato)cobalt(II) and the like. Examples of the magnesium based catalyst include a magnesium chloride such as magnesium dichloride; and the like. Examples of the copper based catalyst include a copper chloride such as copper(I) chloride and copper(II) chloride; and the like. Among these metal catalysts, it is more preferable to use an iron based catalyst. Above all, it is most preferable to use an iron chloride which is excellent in reaction selectivity and which has an effect for advancing the reaction rapidly. Also, such a metal catalyst can be used in an amount of 0.0001 to 0.1 times by mole, and preferably 0.0001 to 0.005 times by mole per mole of the 2,6-dihalogeno-4-trifluoromethylpyridine represented by the formula (II). In addition, in order to obtain the objective material in a high yield, it is preferable to use an iron based catalyst as a metal catalyst. Above all, it is more preferable to use an iron chloride, and it is most industrially preferable to use iron(III) chloride.

Among the 2-halogeno-6-substituted-4-trifluoromethylpyridine represented by the formula (I), a 2-halogeno-6-substituted-4-trifluoromethylpyridine represented by the formula (I-1): 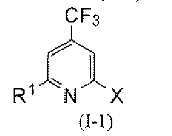 in which, R¹ is alkyl (provided that methyl is excluded), alkenyl, alkynyl, phenyl substituted with alkoxy, benzyl which may be substituted with A, or cycloalkyl; and X and A are as defined above; is a novel compound. Representative examples of these compounds include 2-chloro-6-ethyl-4-trifluoromethylpyridine, 2-chloro-6-propyl-4-trifluoromethylpyridine, 2-chloro-6-isopropyl-4-trifluoromethylpyridine, 2-chloro-6-(cyclopropyl)-4-trifluoromethylpyridine, 2-butyl-6-chloro-4-trifluoromethylpyridine, 2-chloro-6-hexyl-4-trifluoromethylpyridine, 2-chloro-6-(cyclohexyl)-4-trifluoromethylpyridine, 2-benzyl-6-chloro-4-trifluorornethylpyridine, 4-(6-chloro-4-trifluoromethylpyridin-2-yl)anisole, and the like. In addition, as representative examples of the 2,6-dihalogeno-4-trifluoromethylpyridine represented by the formula (II), 2,6-dichloro-4-trifluoromethylpyridine which the present applicant produces and sells, and the like are known.

Also, each of the reagents in the present invention is known, or can be produced by a known method.

The present invention includes the following embodiment, but the present invention is not to be interpreted as being limited thereto.
(1) A process for producing a 2-halogeno-6-suhstituted-4-trifluoromethylpyridine represented by the above formula (I), which comprises allowing a 2,6-dihalogeno-4-trifluoromethylpyridine represented by the above formula (II) and a Grignard reagent represented by the above formula (III) to react with each other in the presence of a solvent.
(2) The process described in the above (1), in which the solvent comprises tetrahydrofuran.
(3) The process described in the above (2), in which the solvent is tetrahydrofuran only.
(4) The process described in the above (2), in which the solvent is a mixture of tetrahydrofuran and an other solvent.
(5) The process described in the above (1), (2), (3) or (4), in which the reaction is carried out in the presence of a metal catalyst.
(6) The process described in the above (5), in which the metal catalyst is an iron based catalyst, a palladium based catalyst, a nickel based catalyst, a zinc based catalyst, a cobalt based catalyst, a magnesium based catalyst, or a copper based catalyst.
(7) The process described in the above (6), in which the metal catalyst is an iron based catalyst.
(8) The process described in the above (7), in which the iron based catalyst is iron chloride, acetylacetonato iron, iron oxide, or metallic iron.
(9) The process described in the above (7), in which the iron based catalyst is iron(II) chloride, iron(III) chloride, bis(acetylacetonato)iron(II), tris(acetylacetonato)iron(III), iron monoxide (FeO), diiron trioxide (Fe₂O₃), triiron tetraoxide (Fe₃O₄), or metallic iron (Fe).
(10) The process described in the above (8), in which the iron based catalyst is an iron chloride.
(11) The process described in the above (10), in which the iron chloride is iron(III) chloride.
(12) The process described in the above (2), in which the reaction temperature is -10°C to a reflux temperature of the solvent.
(13) The process described in the above (2), in which the reaction temperature is-10 to 180°C.
(14) The process described in the above (12), in which the reaction is carried out at the presence of a metal catalyst.
(15) The process described in the above (13), in which the reaction is carried out without the presence of a metal catalyst.
(16) The process described in any one of the above (1) to (15), in which the reaction is carried out under an atmosphere of an inert gas.
(17) The process described in any one of the above (1) to (16), in which the Grignard reagent is methylmagnesium bromide or methylmagnesium chloride.
(18) A 2-halogeno-6-substituted-4-trifluoromethylpyridine represented by the above formula (I-1).
(19) The 2-halogeno-6-substituted-4-trifluoromethylpyridine described in (18), in which the compound represented by the above formula (I-1) is at least one selected from the group consisting of 2-chloro-6-ethyl-4-trifluoromethylpyridine, 2-chloro-6-propyl-4-trifluoromethylpyridine, 2-chloro-6-isoprapyl-4-trifluoromethylpyridine, 2-chloro-6-(cyclopropyl)-4-trifluoromethylpyridine, 2-butyl-6-chloro-4-trifluoromethylpyridine, 2-chloro-6-hexyl-4-trifluoromethylpyridine, 2-chloro-6-(cyclohexyl)-4-trifluoromethylpyridine, 2-benzyl-6-ehloro-4-trifluoromethylpyridine, and 4-(6-chloro-4-trifluoromethylpyridin-2-yl)anisole.

### EXAMPLES

In order to describe the present invention in more detail, Examples are hereunder described, but it should not be construed that the present invention is limited thereto. In each of Synthesis Examples and Table 1, Me represents methyl; Et represents ethyl; n-Pr represents normal propyl; i-Pr represents isopropyl; c-Pr represents cyclopropyl; n-Bu represents normal butyl; n-Hex represents normal hexyl; c-Hex represents cyclohexyl; Bn represents benzyl; Ph represents phenyl; THF represents tetrahydrofuran; and 2,6,4-DCTF represents 2,6-dichloro-4-trifluoromethylpyridine, respectively.

### Synthesis Examples 1

Under a stream of nitrogen gas, 4.62 g (0.0214 mol) of 2,6-dichloro-4-trifluoromethylpyridine and 13 mg of iron(III) chloride as a metal catalyst were dissolved in 20 mL of dry tetrahydrofuran and then, to the resulting solution, 21 mL of a tetrahydrofuran solution of 1.06 mol/L af methylmagnesium bromide (containing 0.0223 mol of MeMgBr) as a Grignard reagent was added dropwise. The mixture was allowed to react at room temperature for 2 hours and then cooled with ice. After 0.6 mL of water was added dropwise, the mixture was stirred at room temperature for a while. The resulting suspension was filtered through celite and then washed three times with dry tetrahydrofuran. Organic layers were combined and then the solution was distilled to obtain 3.08 g (purity: 97.4%, yield: 72%) of 2-ehloro-6-methyl-4-trifluoromethylpyridine as a transparent liquid having a boiling temperature of 140 to 143°C. The measurement results of ¹H-NMR (CDCl₃) of the obtained material were as follows: δ 2.57 (s, 3H), 7.27 (s, 1H), 7.31 (s, 1H). Also, as a result of GC-MS analysis, it was confirmed that the obtained material was an objective material.

### Synthesis Example 2

In the same manner as in Synthesis Example 1, except for using 4.65 g (0.0215 mol) of 2,6-dichloro-4-trifluoromethylpyridine and using 7.5 mL of a tetrahydrofuran solution of 3.0 mol/L of methylmagnesium chloride (containing 0.0226 mol of MeMgCl) as the Grignard reagent, 2.67 g (purity: 95.3%, yield: 61 %) of 2-chloro-6-methyl-4-trifluoromethylpyridine was obtained.

### Synthesis Example 3

In the same manner as in Synthesis Example 1, except for using 4.61 g (0.0213 mol) of 2,6-dichloro-4-trifluoromethylpyridine and using 15 mg of iron(II) chloride as a metal catalyst, 3.16 g (purity: 96.6%, yield: 73%) of 2-chloro-6-methyl-4-trifluoromethylpyridine was obtained.

### Synthesis Example 4

In the same manner as in Synthesis Example 1, except for using 4.61 g (0.0213 mol) of 2,6-dichloro-4-trifduoroxnethylpyridine and using 31 mg of tris(acetylacetonato)iron(III) as a metal catalyst, 2.96 g (purity: 91.2%, yield: 65%) of 2-chloro-6-methyl-4-trifluoromethylpyridine was obtained.

### Synthesis Example 5

In the same manner as in Synthesis Example 1, except for using 4.61 g (0.0213 mol) of 2,6-dichloro-4-trifluoromethylpyridine, using 41 mg of metallic iron as a metal catalyst and carrying out the reaction at room temperature for 24 hours, 3.08 g (purity: 95.4%, yield: 71%) of 2-chloro-6-methyl-4-trifluoromethylpyridine was obtained.

### Synthesis Example 6

In the same manner as in Synthesis Example 1, except for using 113 3 mg of Fe₂O₃ as a metal catalyst and carrying out the reaction at room temperature for 24 hours, 3.11 g (purity: 84.5%, yield: 63%) of 2-chloro-6-methyl-4-trifluoromethylpyridine was obtained.

### Synthesis Example 7

Under a stream of nitrogen gas, 1.33 g (0.00616 mol) of 2,6-dichloro-4-trifluoromethylpyridine and 18 mg of tetrakis(triphenylphosphine)palladium(0) as a metal catalyst were dissolved in 30 mL of dry tetrahydrofuran and then, to the resulting solution, 2.46 mL (0.00739 mol) of a diethyl ether solution of 3.0 mol/L of methylmagnesium bromide as a Grignard reagent was added dropwise. The mixture was allowed to react at room temperature over a day and night and further at a reflux temperature of tetrahydrofuran for 3 hours, followed by cooling with ice. After completion of the reaction, the reaction mixture was poured into cooled dilute hydrochloric acid (one obtained by diluting 0.7 mL of concentrated hydrochloric acid with 50 mL of water), followed by extracting with diethyl ether. After an organic layer was dried over sodium sulfate, the diethyl ether was evaporated. The resulting liquid was purified by distillation. By distillation, 0.546 g (purity: 74.6%, yield: 34%) of a crude product of 2-chloro-6-methyl-4-trifluoromethylpyridine was obtained.

### Synthesis Example 8

Under a stream of nitrogen gas, 4.17 g (0.0193 mol) of 2,6-dichloro-4-trifluoromethylpyridine and 20 mg of iron(III) chloride as a metal catalyst were dissolved in 20 mL of hexane, and then, to the resulting solution, 19.1 mL of a tetrahydrofuran solution of 1.06 mol/L of methylmagnesium bromide (containing 0.0202 mol of MeMgBr) as a Grignard reagent was added dropwise. The mixture was allowed to react at room temperature for 2 hours and then cooled with ice. After 0.7 mL of water was added dropwise, the mixture was stirred at room temperature for a while. The resulting suspension was filtered through celite and then washed three times with dry tetrahydrofuran. Organic layers were combined and the solution was distilled to obtain 2.19 g (purity: 92.0%, yield: 53%) of 2-chloro-6-methyl-4-trifluoromethylpyridine.

### Synthesis Example 9

Under a stream of nitrogen gas, 5.14 g (0.0238 mol) of 2,6-dichloro-4-trifluoromethylpyridine was dissolved in 30 mL of dry tetrahydrofuran, and to the resulting solution, 26.9 mL of a tetrahydrofuran solution of 1.06 mol/L of methylmagnesium bromide (containing 0.0286 mol of MeMgBr) as a Grignard reagent was added dropwise. The mixture was allowed to react at 67°C for 17 hours and then cooled with ice. The reaction mixture was poured into cold water and then stirred at room temperature for a while. The resulting suspension was filtered through celite and then extracted three times with diethyl ether. The solvent was evaporated, and the residue was distilled to obtain 2.95 g (purity: 78.4%, yield: 50%) of a crude product of 2-chloro.6-methyl-4-trifluoromethylpyridinc.

### Synthesis Examples 10

To 20 mL of dry tetrahydrofuran having 4.61 g (0.0213 mol) of2,6-dichloro-4-trifluoromethylpyridine and 16 mg of iron(III) chloride as a metal catalyst dissolved therein, 22.4 mL of a tetrahydrofuran solution of 1.00 mol/L of methylmagnesium bromide (containing 0.0224 mol of EtMgBr) as a Grignard reagent was added dropwise under a stream of nitrogen gas below 15°C. The mixture was allowed to react at room temperature for 3 hours and then cooled with ice. After water (0.7 mL) was added dropwise, the mixture was stirred at room temperature for 3.5 hours. The resulting suspension was filtered through celite and then washed three times with dry tetrahydrofuran (total amount: 40 mL). Organic layers were combined and the solution was distilled to obtain 3.04 g (purity: 64.3%, yield: 44%) of a crude product of 2-chloro-6-ethyl-4-trifluoromethylpyridine as a transparent liquid having a boiling temperature of 148.0 to 151.5 °C. The measurement results of ¹H-NMR (CDCl₃) of the obtained material were as follows: δ 1.28 (t, 3H), 2.83(q, 2H), 7.24 (s, 1H), 7.32 (s, 1H). In addition, as a result of GC-MS analysis, it was confirmed that the obtained product was the objective compound.

### Synthesis Example 11

In the same manner as in Synthesis Example 10, 2.28 g (purity: 84.9%, yield: 43%) of 2-chloro-6-ethyl-4-trifluoromethylpyridine was obtained, except for using 31.4 mL of a tetrahydrofuran solution of 1.00 mol/L of ethylmagnesium bromine (containing 0.0314 mol of EtMgBr) as a Grignard reagent, using 17 mg of iron(III) chloride as the metal catalyst and carrying out the reaction at room temperature for 1.3 hours.

### Synthesis Example 12

To 20 mL of dry tetrahydrofuran having 4.62 g (0.0214 mol) of 2,6-dichloro-4-trifluoromethylpyridine and 17 mg of iron(III) chloride as a metal catalyst dissolved therein, 25.0 mL of a tetrahydrofuran solution of 1.04 mol/L of n-propylrnagnesiurn bromide (containing 0.0260 mol ofn-PrMgBr) as a Grignard reagent was added dropwise under a stream of nitrogen gas below 16°C, and the mixture was then allowed to react at room temperature for one hour. Since the reactant remained, 5.0 mL of a tetrahydrofuran solution of 1.04 mol/L of n-propylmagnesium bromide (containing 0.0052 mol of n-PrMgBr) was further added. The mixture was allowed to react at room temperature for one hour and then cooled with ice. After cold water (0.7 mL) was added dropwise, the mixture was stirred at room temperature for 2 hours. The resulting suspension was filtered through celite and then washed three times with dry tetrahydrofuran (total amount: 50 mL). Organic layers were combined and distilled to obtain 2.77 g (purity: 84.8%, yield: 49%) of 2-chloro-6-propyl-4-trifluoromethylpyridine as a transparent liquid having a boiling temperature of 162 to 165°C. The measurement results of ¹H-NMR (CDCl₃) of the obtained material were as follows: δ 0.92 (t, 3H), 1.68 (dt, 2H), 2.78 (t, 2H), 7.24 (s, 1H), 7.36 (s, 1H). In addition, as a result of GC-MS analysis, it was confirmed that the obtained product was the objective compound.

### Synthesis Examples 13

To 20 mL) of dry tetrahydrofuran having 4.62 g (0.0214 mol) of 2,6-dichloro-4-trifluoromethylpyridine and 15 mg of iron(III) chloride as a metal catalyst dissolved therein, 25.7 mL of a tetrahydrofuran solution of 1.0 mol/L of cyclopropylmagnesium bromide (containing 0.0257 mol of c-PrMgBr) as a Grignard reagent was added dropwise under a stream of nitrogen gas below 16°C. Since the reactant remained, 10.0 mL of a tetrahydrofuran solution of 1.0 mol/L of cyclopropylmagnesium bromide (containing 0.0100 mol of c-PrMgBr) was further added below 13°C. The mixture was allowed to react at room temperature for 2 hours and then cooled with ice. After 1.2 mL of water was added dropwise, the mixture was stirred at room temperature for 2 hours. The resulting suspension was filtered through celite and then washed three times with dry tetrahydrofuran (total amount: 50 mL). Organic layers were combined and distilled to obtain 3.72 g (purity: 89.6%, yield: 70%) of 2-chloro-6-(cyclopropyl)-4-trifluoromethylpyridine as a transparent liquid having a boiling temperature of 167 to 175°C. The measurement results of ¹H-NMR (CDCl₃₎ of the obtained material were as follows: δ 0.76-0.80 (m, 4H), 1.70-1.76 (m, 1H), 6.93 (s, 1H), 6.95 (s, 1H). In addition, as a result of GC-MS analysis, it was confirmed that the obtained product was the objective compound.

### Synthesis Example 14

To 20 mL of dry tetrahydrofuran into which 4.61 g (0.0213 mol) of 2,6-dichloro-4-trifluorozrzethylpyridine and 17 mg of iron(III) chloride as a metal catalyst dissolved, 11.18 mL of a tetrahydrofuran solution of 2.00 mol/L of n-butylmagnesium chloride (containing 0.0224 mol of n-BuMgCl) as a Grignard reagent was added dropwise under a stream of nitrogen gas below 15°C over 30 minutes. The mixture was allowed to react at room temperature for 2 hours and then cooled with ice. After cold water (0.7 mL) was added dropwise, the mixture was stirred at room temperature for 3 hours. The resulting brown suspension was filtered through celite and then washed with dry tetrahydrofuran (total amount: 40 mL). Organic layers were combined and distilled to obtain 3.44 g (purity: 84.5%, yield: 57%) of2-butyl-6-chloro-4-trifluoromethylpyridine as a transparent liquid having a boiling temperature of 165 to 179°C. The measurement results of ¹H-NMR (CDCl₃) of the obtained material were as follows: δ 0.89 (t, 3H), 1.33-1.40 (m, 2H), 1.66-1.71 (m, 2H), 2.80 (t, 2H), 7.24 (s, 1H), 7.33 (s, 1H). In addition, as a result of GC-MS analysis, it was confirmed that the obtained product was the objective compound.

### Synthesis Example 15

To 20 mL of dry tetrahydrofuran into which 4.61 g (0.0213 mol) of 2,6-dichloro-4-trifluoromethylpyridine and 15 mg of iron(III) chloride as a metal catalyst dissolved, 12.8 mL of a tetrahydrofuran solution of 2.0 mol/L of hexylmagnesium chloride (containing 2.56×10⁻² mol of n-HexMgCl) as a Grignard reagent was added dropwise under a stream of nitrogen gas below 21°C. Since the reactant remained, 1.20 mL (2.40×10⁻³ mol) of a hexylmagnesium chloride solution was added below 13°C. The mixture was allowed to react at room temperature for 2 hours and then cooled with ice. After cold water (1.0 mL) was added dropwise below 13°C, the mixture was stirred at room temperature for 2 hours. The resulting brown suspension was filtered through celite and then washed with dry tetrahydrofuran (total amount: 40 mL). After organic layers were combined and concentrated, the resulting blackish brown liquid was distilled to obtain 0.847 g (purity: 74.2%) of a transparent liquid having a boiling temperature of 105 to 118°C/32 hPa and 3.73 g (purity: 90.1%, overall yield: 70%) of a transparent liquid having a boiling temperature of 118 to 128°C/32 hPa. The measurement results of ¹H-NMR (CDCl₃) of the both transparent liquids were as follows: δ 0.82-0.86 (m, 3H), 1.25-1.42 (m, 6H), 1.66-1.81 (m, 2H), 2.80 (t, 2H), 7.24 (s, 1H), 7.33 (s, 1H). Also, as a result of GC-MS analysis, it was confirmed that the both transparent liquids were 2-chloro-6-hexyl-4-tritluoromethylpyridine.

### Synthesis Example 16

To 20 mL of dry tetrahydrofuran into which 4.62 g (0.0214 mol) of 2,6-dichloro-4-trifluoroznethylpyridine and 18 mg of iron(III) chloride as a metal catalyst dissolved, 26.0 mL of a tetrahydrofuran solution of 1.0 mol/L of cyclohexylmagnesium bromide (containing 0.026 mol of c-HexMgBr) as a Grignard reagent was added dropwise under a stream of nitrogen gas below 21°C, and the mixture was then allowed to react for 40 minutes. Since the reactant remained, 8.0 mL of a tetrahydrofuran solution of 1.0 mol/L of cyc10hexylmagnesium bromide (containing 0.008 mol of c-HexMgBr) was added below 20°C. The mixture was allowed to react at room temperature for 2 hours and then cooled with ice. After 1.2 mL of water was added dropwise below 18°C, the mixture was stirred at room temperature for 2 hours. The resulting suspension was filtered through celite and then washed with dry tetrahydrofuran (total amount: 40 mL). Organic layers were combined and distilled to obtain 3.19 g (purity: 93.2%, yield: 53%) of 2-chloro-6-(cyclohexyl)-4-trifluoromethylpyridine as a transparent liquid having a boiling temperature of 120 to 132°C/30 to 34 hPa. The measurement results of ¹H-NMR (CDCl₃) of the obtained material were as follows: δ 1.22-1.95 (m, 10H), 2.70-2.76 (m, 1H), 7.24 (s, 1H), 7.32 (s, 1H). In addition, as a result of GC-MS analysis, it was confirmed that the obtained product was the objective compound.

### Synthesis Example 17

To dry tetrahydrofuran (20 mL) into which 4.62 g (0.0214 mol) of 2,6-dichloro-4-trifluoromethylpyridine and 17 mg of iron(III) chloride as a metal catalyst dissolved, 24.5 mL of a tetrahydrofuran solution of 0.93 mol/L of benzylmagnesium chloride (containing 0.0228 mol of BnMgCl) as a Grignard reagent was added dropwise under a stream of nitrogen gas below 18°C. The mixture was allowed to react at room temperature for 2 hours and then cooled with ice. After water (0.7 mL) was added dropwise below 13°C, the mixture was stirred at room temperature for 1.5 hours. The resulting suspension was filtered through celite and then washed with dry tetrahydrofuran (total amount: 40 mL). Organic layers were combined and concentrated, and the resulting brown liquid was purified by column chromatography (ethyl acetate/hexane = 5/95 to 1/9), to obtain 4.99 g (purity: 80.4%, yield: 69%) of 2-benzyl-6-chloro-4-trifluoromethylpyridine as a yellow liquid. The measurement results of ¹H-NMR (CDCl₃) of the obtained material were as follows: δ 4.16 (s, 2H), 7.21 (s, 1H), 7.21-7.32 (m, 5H), 7.35 (s, 1H). In addition, as a result of GC-MS analysis, it was confirmed that the obtained product was the objective compound.

### Synthesis Examples 18

To 20 mL of dry tetrahydrofuran into which 4.61 g (0.0213 mol) of 2,6-dichloro-4-trifluoromethylpyridine and 17 mg of iron(III) chloride as a metal catalyst dissolved, 44.73 mL of a tetrahydrofuran solution of 0.5 mol/L of 4-methoxyphenylmagnesium bromide (containing 0.0224 mol of p-MeO-PhMgBr) as a Grignard reagent is added dropwise under a stream of nitrogen gas below 7°C. The mixture is allowed to react at room temperature for 6.5 hours and then cooled with ice. After 0.7 mL of water is added dropwise, the mixture is stirred at room temperature for one hour. The thus obtained suspension is filtered through celite and washed with dry tetrahydrofuran. Organic layers are combined and concentrated, and the resultant is purified by column chromatography (ethyl acetate/hexane = 5/95 to 6/4), to obtain 4-(6-chloro-4-trifluoromethylpyridin-2-yl)anisole.

The reaction condition, amount obtained, purity and yield of each of the foregoing Synthesis Examples 1 to 17 were summarized in the following Table 1. Also, a molar ratio of each of the Grignard reagent and the metal catalyst per mole of 2,6-dichloro-4-trifluoromethylpyridinc (2,6,4-DCTF) was shown together in the parenthesis in Table 1.
[Table 1]

**Table 1**

| Synthesis Example | 2,6,4-DCTF (Molar ratio) | Total amount of Grignard reagent (Molar ratio) | Metal catalyst (Molar ratio) | Solvent | Reaction temperature × Time | Amount obtained (Purity) Yield |
|---|---|---|---|---|---|---|
| 1 | 4.62g | MeMgBr | FeCl₃ | THF | Room temperature | 3.08g |
| | 0.0214 mol | 0.0223 mole | 13 mg | | × 2 hours | (97.4%) |
| | (1) | (1.04) | (0.0037) | | | 72% |
| 2 | 4.65g | MeMgCl | FeCl₃ | THF | Room temperature | 2.67g |
| | 0.0215 mol | 0.0226 mol | 13 mg | | × 2 hours | (95.3%) |
| | (1) | (1.05) | (0.0038) | | | 61% |
| 3 | 4.61 g | MeMgBr | FeC1₂ | THF | Room temperature | 3.16g |
| | 0.0213 mol | 0.0223 mol | 15 mg | | × 2 hours | (96.6%) |
| | (1) | (1.05) | (0.0056) | | | 73% |
| 4 | 4.6 1 g | MeMgBr | Tris(acetylacetonato) | THF | Room temperature | 2.96g |
| | 0.0213 mol | 0.0223 mol | iron (III) | | × 2 hours | (91.2%) |
| | (1) | (1.05) | 31 mg | | | 65% |
| | | | (0.0041) | | | |
| 5 | 4.61g | MeMgBr | Fe | THF | Room temperature | 3.08g |
| | 0.0213 mol | 0.0223 mol | 41 mg | | × 24 hours | (95.4%) |
| | (1) | (1.05) | (0.0345) | | | 71% |
| 6 | 4.62g | MeMgBr | Fe₂O₃ | THF | Room temperature | 3.11g |
| | 0.0214 mol | 0.0223 mol | 113 mg | | × 24 hours | (84.5%) |
| | (1) | (1.05) | (0.0337) | | | 63% |
| 7 | 1.33g | MeMgBr | Pd(PPh₃)₄ | THF | Room temperature | 0.546g |
| | 0.00616 mol | 0.00739 mol | 18 mg | | × All day | (74.6%) |
| | (1) | (1.20) | (0.0025) | | Reflux temperature | 34% |
| | | | | | × 3 hours | |
| 8 | 4.17g | MeMgBr | FeCl₃ | Hexan | Room temperature | 2.19g |
| | 0.0193 mol | 0.0202 mol | 20 mg | | × 2 hours | (92.0%) |
| | (1) | (1.05) | (0.0064) | | | 53% |
| 9 | 5.14g | MeMgBr | Absent | THF | 67°C | 2.95g |
| | 0.0238 mol | 0.0286 mol | | | × 17 hours | (78.4%) |
| | (1) | (1.20) | | | | 50% |
| 10 | 4.61g | EtMgBr | FeCl₃ | THF | Room temperature | 3.04g |
| | 0.0213 mol | 0.0224 mol | 16 mg | | ×3 hours | (64.3%) |
| | (1) | (1.05) | (0.0046) | | | 44% |
| 11 | 4.61g | EtMgBr | FeCl₃ | THF | Room temperature | 2.28g |
| | 0.0213 mol | 0.0314 mol | 17 mg | | × 1.3 hours | (84.9%) |
| | (1) | (1.47) | (0.0049) | | | 43% |
| 12 | 4.62g | n-PrMgBr | FeCl₃ | THF | Room temperature | 2.77g |
| | 0.0214 mol | 0.0312 mol | 17 mg | | × 2 hours | (84.8%) |
| | (1) | (1.46) | (0.0049) | | | 49% |

**[Table 2]**

| Table 1 (Continued) | | | | | | |
|---|---|---|---|---|---|---|
| Synthesis Example | 2,6,4-DCTF (Molar ratio) | Total amount of Grignard reagent (Molar ratio) | Metal catalyst (Molar ratio) | Solvent | Reaction temperature × Time | Amount obtained (Purity) Yield |
| 13 | 4.62g | c-PrMgBr | FeCl₃ | THF | Room temperature | 3.72g |
| | 0.0214 mol | 0.0357 mol | 15 mg | | × 2 hours | (89.6%) |
| | (1) | (1.67) | (0.0043) | | | 70% |
| 14 | 4.6 1 g | n-BuMgCl | FeCl₃ | THF | Room temperature | 3.44g |
| | 0.0213 mo1 | 0.0224 mol | 17 mg | | × 2 hours | (84.5%) |
| | (1) | (1.05) | (0.0049) | | | 57% |
| 15 | 4.61g | n-HexMgCl | FeCl₃ | THF | Room temperature | 0.847g |
| | 0.0213 mol | 0.0280 mol | 15 mg | | × 2 hours | (74.2%)+ |
| | (1) | (1.31) | (0.0043) | | | 3.73g |
| | | | | | | (90.1%) |
| | | | | | | Total yield 70% |
| 16 | 4.62g | c-HexMgBr | FeCl₃ | THF | Room temperature | 3.19g |
| | 0.0214 mol | 0.034mol | 18mg | | x 3 hours | (93.2%) |
| | (1) | (1.59) | (0.0052) | | | 53% |
| 17 | 4.62g | BnMgCl | FeCl₃ | THF | Room temperature | 4.99g |
| | 0.0214 mol | 0.0228 mol | 17 mg | | × 2 hours | (80.4%) |
| | (1) | (1.06) | (0.0049) | | | 69% |

While the present invention has been described in detail and with reference to specific embodiments thereof, it will be apparent to one skilled in the art that various changes and modifications can be made therein without departing from the spirit and scope thereof.

This application is based on the Japanese patent application filed on April 17, 2009 (Japanese Patent Application No. 2009-100749) and the entire contents of which are incorporated hereinto by reference. All references cited herein are incorporated in their entirety.

### INDUSTRIAL APPLICABILITY

According to the producing process of the present invention, the 2-halogeno-6-substituted-4-trifluoromethylpyridine can be produced by selectively replacing only one of the halogens of the 2,6-dihalogeno-4-trifluoromethylpyridirle by another substituent. Also, the objective material can be produced in a high yield by selecting tetrahydrofuran as a solvent. Furthermore, the objective material can be produced in a high yield even at room temperature by using a specific metal catalyst in combination.

## Claims

1. A process for producing a 2-halogeno-6-substittited-4-trifluoromethylpyridine represented by the formula (I): 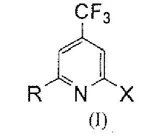 wherein X is a chlorine atom, a bromine atom, or an iodine atom; R is alkyl, alkenyl, alkynyl, phenyl which may be substituted with A, benzyl which may be substituted with A, or cycloalkyl; and A is alkyl, alkoxy, a fluorine atom, or a chlorine atom,
which comprises allowing a 2,6-dihalogeno-4-trifluoromethylpyridne represented by the formula (II): 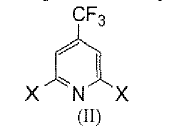 wherein X is as defined above,
and a Grignard reagent represented by the formula (III): RMgX, in which R and X are defined above, to react with each other in the presence of a solvent.

2. The process according to claim 1, wherein the solvent comprises tetrahydrofuran.

3. The process according to claim 1 or 2, wherein the reaction is carried out in the presence of a metal catalyst.

4. The process according to claim 3, wherein the metal catalyst is an iron based catalyst, a palladium based catalyst, a nickel based catalyst, a zinc based catalyst, a cobalt based catalyst, a magnesium based catalyst, or a copper based catalyst.

5. The process according to claim 4, wherein the metal catalyst is an iron based catalyst.

6. The process according to claim 5, wherein the iron based catalyst is iron chloride, acetylacetonato iron, iron oxide, or metallic iron.

7. The process according to claim 6, wherein the iron based catalyst is iron chloride.

8. The process according to claim 1, wherein the Grignard reagent is methylmagnesium bromide or methylmagnesium chloride.

9. A 2-halogeno-6-substituted-4-trifluoromethylpyridine represented by the formula (I-1): 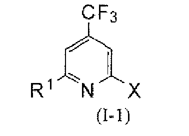 wherein X is a chlorine atom, a bromine atom, or an iodine atom; R¹ is alkyl (provided that methyl is excluded), alkenyl, alkynyl, phenyl substituted with alkoxy, benzyl which may be substituted with A, or cycloalkyl; and A is alkyl, alkoxy, a fluorine atom, or a chlorine atom.

10. The 2-halogeno-6-substituted-4-trifluoromethylpyridine according to claim 9, which is at least one selected from the group consisting of 2-chloro-6-ethyl-4-trifluoromethylpyridine, 2-chloro-6-propyl-4-trifluoromethylpyridine, 2-chloro-6-isopropyl-4-trifluoromethylpyridine, 2-chloro-6-(cyclopropyl)-4-trifluoromethylpyridine, 2-butyl-6-chloro-4-trifluoroznethylpyridine, 2-chloro-6-hexyl-4-trifluoromechylpyridine, 2-chloro-6-(cylohexyl)-4-trifluoromcthylpyridine, 2-benzyl-6-clzloro-4-trifluoromethylpyridine, and 4-(6-chloro-4-trifluorornethylpyridin-2-yl)anisole.
